# EUROPEAN PATENT SPECIFICATION

(11) **EP 3 332 801 B1**
(45) Date of publication and mention of the grant of the patent: **13.09.2023**
(21) Application number: 17191973.1
(22) Date of filing: 12.03.2009
(51) Int. Cl.: A61K 39/00, A61P 37/06

(54) **DNA VACCINES AND METHODS FOR THE PREVENTION OF TRANSPLANTATION REJECTION**
DNA-IMPFSTOFFE UND VERFAHREN ZUR VORBEUGUNG DER TRANSPLANTATIONSABSTOSSUNG
VACCINS D'ADN ET PROCÉDÉS POUR LA PRÉVENTION DU REJET DE TRANSPLANTATION

(30) Priority: 12.03.2008 US 36004 P
(43) Date of publication of application: 13.06.2018
(62) Divisional of application: 09719482.3
(73) Proprietor: LOMA LINDA UNIVERSITY, Loma Linda, CA 92350 (US)
(72) Inventor: LI, Fengchun, Loma Linda, CA 92354-2732 (US); ESCHER, Alan P., Redlands, CA 92374 (US)
(74) Representative: Gregson, Anna Louise

(56) References cited:
- WO-A2-2006/124375
- LI A ET AL: "A therapeutic DNA vaccination strategy for autoimmunity and transplantation", VACCINE, ELSEVIER LTD, GB, vol. 28, no. 8, 23 February 2010 (2010-02-23), pages 1897-1904, XP026921808, ISSN: 0264-410X, DOI: 10.1016/J.VACCINE.2009.10.090 [retrieved on 2010-02-25]
- LI A F ET AL: "DNA vaccines for transplantation", EXPERT OPINION ON BIOLOGICAL THERAPY, ASHLEY, LONDON, GB, vol. 10, no. 6, 1 June 2010 (2010-06-01), pages 903-915, XP009167980, ISSN: 1471-2598
- SHABAHANG SHAHROKH ET AL: "Recent patents on immunoregulatory DNA vaccines for autoimmune diseases and allograft rejection.", RECENT PATENTS ON DNA & GENE SEQUENCES JUN 2010, vol. 4, no. 2, June 2010 (2010-06), pages 122-131, XP009168337, ISSN: 2212-3431
- LI A F ET AL: "Co-delivery of pro-apoptotic BAX with a DNA vaccine recruits dendritic cells and promotes efficacy of autoimmune diabetes prevention in mice", VAC, ELSEVIER, AMSTERDAM, NL, vol. 22, no. 13-14, 16 April 2004 (2004-04-16), pages 1751-1763, XP004500430, ISSN: 0264-410X, DOI: 10.1016/J.VACCINE.2003.10.049
- ANDRES ET AL: "Cancer incidence after immunosuppressive treatment following kidney transplantation", CRITICAL REVIEWS IN ONCOLOGY/HEMATOLOGY, ELSEVIER, AMSTERDAM, NL, vol. 56, no. 1, 1 October 2005 (2005-10-01), pages 71-85, XP027789931, ISSN: 1040-8428 [retrieved on 2005-10-01]
- YU X Z ET AL: "Advances in transplantation tolerance", THE LANCET (LETTERS TO THE EDITOR), THE LANCET PUBLISHING GROUP, GB, vol. 357, no. 9272, 16 June 2001 (2001-06-16), pages 1959-1963, XP004800667, ISSN: 0140-6736, DOI: 10.1016/S0140-6736(00)05068-6
- VALUJSKIKH ANNA ET AL: "Development of autoimmunity after skin graft rejection via an indirect alloresponse", TRANSPLANTATION (BALTIMORE), vol. 73, no. 7, 15 April 2002 (2002-04-15) , pages 1130-1137, XP009504947, ISSN: 0041-1337
- LI A ET AL: "T.80. DNA Vaccination for Type 1 Diabetes Prolongs Skin Allograft Survival in a Donor-Specific Manner", CLINICAL IMMUNOLOGY, ACADEMIC PRESS, US, vol. 131, 1 January 2009 (2009-01-01), page S76, XP026084615, ISSN: 1521-6616, DOI: 10.1016/J.CLIM.2009.03.222 [retrieved on 2009-01-01]

## Description

### BACKGROUND

Prevention of organ rejection in the clinic presently relies on administration of cocktails of immunosuppressants (Tacrolimus, Rapamycin, MMF, AZA, corticosteroids). The drugs are efficient for prevention of acute rejection, but do not prevent chronic rejection. In addition, because these drugs interfere with immune responses non-specifically, their chronic use exposes patients to high risks of cancer and infection. Other approaches that are being tested are co-stimulatory blockade and bone-marrow chimerism. However, "late-onset chronic rejection, as well as the toxicity of some of these regimens, remain as significant limitations that hamper clinical application" (Ochiai et al., Front Biosci. 2007, 12:4248-53).

WO 2006/124375 A2 describes substances, compositions and methods for preventing and treating immune-related inflammatory disorders.

Li et al. (Vaccine. 2004 Apr 16;22(13-14):1751-63) teaches that co-delivery of pro-apoptotic BAX with a DNA vaccine recruits dendritic cells and promotes efficacy of autoimmune diabetes prevention in mice.

Andres et al. (Crit Rev Oncol Hematol. 2005 Oct;56(1):71-85) describes cancer incidence after immunosuppressive treatment following kidney transplantation.

Yu et al. (Lancet. 2001 Jun 16;357(9272):1959-63) describes advances in transplantation tolerance.

Valujskikh et al. (Transplantation. 2002 Apr 15;73(7):1130-7) describes the development of autoimmunity after skin graft rejection via an indirect alloresponse.

Li et al. (Clinical Immunology, Academic Press. 2009 Jan 2009 (131):576) describes how DNA vaccination for Type I diabetes prolongs skin allograft survival in a donor-specific manner.

### SUMMARY

The invention is defined by the claims. Those aspects/instances of the present disclosure which constitute the invention are defined by the claims.

The present invention provides a DNA vaccine for use in preventing, delaying the onset of, or treating rejection of a skin allograft or a skin allogeneic transplant, wherein the DNA vaccine comprises (a) a first plasmid comprising a polynucleotide encoding secreted glutamic acid decarboxylase 55 (sGAD55) under the control of a promoter which expresses the polynucleotide encoding sGAD55, and comprising a plurality of CpG motifs, wherein the CpG motifs are methylated sufficiently to diminish the recipient's immune response to unmethylated CpG motifs, and wherein at least some of the plurality of CpG motifs are methylated and resistant to digestion by restriction endonuclease HpaII, and (b) a second plasmid comprising a polynucleotide encoding Bax under the control of a promoter which expresses the polynucleotide encoding BAX; and wherein the use comprises:
(i) selecting a recipient in need of a graft or transplant and an allograft or allogeneic transplant donor;
(ii) grafting tissue or transplanting skin from the donor to the recipient; and
(iii) intradermally administering to the recipient one or more than one dose of the DNA vaccine.

In one embodiment the CpG motifs of one or more than one plasmid are methylated by Sssl methylase.

In one embodiment of the present invention, the promoter capable of expressing the polynucleotide encoding the autoantigen or the donor antigen (i.e., secreted glutamic acid decarboxylase 55 (sGAD55)), or the promoter capable of expressing the polynucleotide encoding the pro-apoptotic protein (i.e., BAX), or both the promoter capable of expressing the polynucleotide encoding the autoantigen or the donor antigen, and the promoter capable of expressing the polynucleotide encoding the pro-apoptotic protein maintain their promoter function after methylation.

In one embodiment of the present invention, the DNA vaccine comprises the first plasmid and the second plasmid in a ratio of between 1/1000 to 1000/1. In a preferred embodiment, the DNA vaccine comprises the first plasmid and the second plasmid in a ratio of between 1/100 to 100/1. In a particularly preferred embodiment, the DNA vaccine comprises the first plasmid and the second plasmid in a ratio of between 1/10 to 10/1.

In one embodiment the DNA vaccine is administered in an effective dose, wherein an effective dose is an amount sufficient to prevent, delay the onset, or treat rejection of an allograft or an allogeneic transplant by the recipient.

In another embodiment, the DNA vaccine is administered in an effective dose, wherein an effective dose is an amount sufficient to induce a donor- specific tolerogenic response.

In preferred embodiments, the DNA vaccine is (a) administered in a plurality of doses; (b) the dose of the DNA vaccine is about 0.001 mg/Kg of body weight of the recipient to about 100 mg/Kg of body weight of the recipient; and/or (c) the dose is administered weekly between two times and 100 times.

In some embodiments the DNA vaccine is administered by an injection proximal to the site of the allograft or allogeneic transplant.

In one embodiment, the DNA vaccine is for use in a method comprising the step of administering a dose of one or more than one immunosuppressant agent before, on the day of and/or after engraftment or transplantation. As will be appreciated by one of skill in the art, with reference to the present disclosure, the dose of one or more than one immunosuppressant agent can be administered simultaneously, separately or sequentially.

In specific embodiments, the one or more than one immunosuppressant agent is selected from the group consisting of corticosteroids, glucocorticoids, cyclophosphamide, 6-mercaptopurine, azathioprine, methotrexate cyclosporine, mycophenolate mofetil, mycophenolic acid, tacrolimus, sirolimus, everolimus, mizoribine, leflunomide, deoxyspergualin, brequinar, azodicarbonamide, vitamin D analogs, antilymphocyte globulin , antithymocyte globulin, anti-CD3 monoclonal antibodies, anti-interleukin-2 receptor (anti-CD25) antibodies, anti-CD52 antibodies, anti-CD20 antibodies, anti-tumor necrosis factor reagents and LFA-1 inhibitors.

In a preferred embodiment, the method includes the step of administering a single dose of antilymphocyte globulin, at a dosage of about 1.6 mg/20g of body weight, on the day of engraftment or transplantation.

In another preferred embodiment, the method includes the step of administering rapamycin at a dosage of from 0.05 to 15 mg/day.

### DETAILED DESCRIPTION

One aspect of the present disclosure comprises a method for preventing, delaying the onset of, or treating rejection of an allograftor an allogeneic transplant. The method comprises: (A) selecting a recipient in need of a graft or transplant and an allograftorallogeneic transplant donor; (B) grafting tissue or transplanting a solid organ from the donor to the recipient; and (C) administering to the recipient one or more than one dose of a DNA vaccine. The DNA vaccine is selected from the group consisting of: three individual plasmids and a two plasmid vaccine. The first plasmid comprises (a) a polynucleotide encoding a pro-apoptotic protein; and (b) a promoter controlling the expression of the polynucleotide encoding the pro-apoptotic protein. The second plasmid comprises: (a) a polynucleotide sequence encoding an autoantigen or donor antigen; (b) a promoter controlling expression of the polynucleotide sequence encoding the autoantigen or donor antigen; and (c) a plurality of CpG motifs, where the CpG motifs are methylated sufficiently to diminish the recipient's immune response to unmethylated CpG motifs. The third plasmid comprises: (a) a polynucleotide sequence encoding an autoantigen or donor antigen; (b) a promoter controlling expression of the polynucleotide sequence encoding the autoantigen or donor antigen; (c) a polynucleotide encoding a pro-apoptotic protein; (d) a promoter controlling expression of the polynucleotide encoding the pro-apoptotic protein; and (e) a plurality of CpG motifs, where the CpG motifs are methylated sufficiently to diminish the recipient's immune response to unmethylated CpG motifs. The two plasmid vaccine comprises a combination of the first plasmid and the second plasmid.

In some instances, the engrafted tissues or transplanted organs are selected from the group consisting of skin grafts, islet cell transplants, and partial or whole organ transplants. In additional instances,
partial or whole organ transplants are selected from the group consisting of hearts, lungs, kidneys and livers.

In one instance, the DNA vaccine is one or more than one plasmid comprising a plurality of methylated CpG motifs, where the one or more than one plasmid is resistant to digestion by the restriction enzyme HpaII. In a preferred instance, the DNA vaccine is one or more than one plasmid comprising a plurality of CpG motifs, where the CpG motifs of one or more than one plasmid are methylated by SssI methylase.

In one instance, the promoter capable of expressing the polynucleotide encoding the autoantigen or the donor antigen, or the promoter capable of expressing the polynucleotide encoding the pro-apoptotic protein, or both the promoter capable of expressing the polynucleotide encoding the autoantigen or the donor antigen, and the promoter capable of expressing the polynucleotide encoding the pro-apoptotic protein maintain their promoter function after methylation.

In another instance, the third plasmid further comprises an internal ribosome entry site (IRES) sequence, where the IRES sequence is SEQ ID NO:3 from the EMC virus, to permit translation of the polynucleotide encoding the autoantigen or the donor antigen, and the polynucleotide encoding the pro-apoptotic protein from the same transcript.

In one instance, the DNA vaccine comprises the first plasmid and the second plasmid in a ratio of between 1/1000 to 1000/1. In a preferred instance, DNA vaccine comprises the first plasmid and the second plasmid in a ratio of between 1/100 to 100/1. In a particularly preferred instance, the DNA vaccine comprises the first plasmid and the second plasmid in a ratio of between 1/10 to 10/1.

In specific instances, the autoantigen or donor antigen is selected from the group consisting of carbonic anhydrase II, collagen, CYP2D6 (cytochrome P450, family 2, subfamily Device 400, polypeptide 6), glutamic acid decarboxylase, secreted glutamic acid decarboxylase 55, SEQ ID NO:1, insulin, myelin basic protein and SOX-10 (SRY-box containing gene 10) or any relevant autoantigen that is present in both the transplant recipient and the donor allograft.

In additional specific instances, the pro-apoptotic protein is selected from the group consisting of BAX, SEQ ID NO:2, a modified caspase, Tumor Necrosis Factor Receptor, Death Receptor 3 (DR3), Death Receptor 4 (DR4), Death Receptor 5 (DR5) and a FAS receptor.

In one instance, the DNA vaccine is administered in an effective dose, wherein an effective dose is an amount sufficient to prevent, delay the onset, or treat rejection of an allograft or an allogeneic transplant by the recipient.

In another instance, the DNA vaccine is administered in an effective dose, wherein an effective dose is an amount sufficient to induce a donor-specific tolerogenic response.

In preferred instances, the DNA vaccine is (a) administered in a plurality of doses; (b) the dose of the DNA vaccine is about 0.001 mg/Kg of body weight of the recipient to about 100 mg/Kg of body weight of the recipient; and/or (c) the dose is administered weekly between two times and 100 times.

In some instances, the DNA vaccine is administered by an epidermal, intradermal, intramuscular, intranasal, intravenous, intraperitoneal or oral route. In a preferred instance, the DNA vaccine is administered by an injection proximal to the site of the allograft or allogeneic transplant.

In one instance, the DNA vaccine is for use in a method comprising the step of administering a dose of one or more than one immunosuppressant agent before, on the day of and/or after engraftment or transplantation. As will be appreciated by one of skill in the art, with reference to the present disclosure, the dose of one or more than one immunosuppressant agent can be administered simultaneously, separately or sequentially.

In specific instances, the one or more than one immunosuppressant agent is selected from the group consisting of corticosteroids, glucocorticoids. cyclophosphamide, 6-mercaptopurine, azathioprine, methotrexate cyclosporine, mycophenolate mofetil, mycophenolic acid, tacrolimus, sirolimus, everolimus, mizoribine, leflunomide, deoxyspergualin, brequinar, azodicarbonamide, vitamin D analogs, antilymphocyte globulin , antithymocyte globulin, anti-CD3 monoclonal antibodies, anti-interleukin-2 receptor (anti-CD25) antibodies, anti-CD52 antibodies, anti-CD20 antibodies, anti-tumor necrosis factor reagents and LFA-1 inhibitors.

In a preferred instance, the method includes the step of administering a single dose of antilymphocyte globulin, at a dosage of about 1.6 mg/20g of body weight, on the day of engraftment or transplantation.

In another preferred instance, the method includes the step of administering rapamycin at a dosage of from 0.05 to 15 mg/day.

One instance provides a method for preventing, delaying the onset of or treating rejection of an allograft or an allogeneic transplant, comprising the steps of: (A) selecting a graft or transplant recipient and an allograft or allogeneic transplant donor; (B) grafting tissue or transplanting a solid organ from the donor to the recipient; and (C) inducing a donor-specific immune response that elevates regulatory T cell activity by administering to the recipient one or more than one dose of a DNA vaccine comprising a first plasmid and a second plasmid. The first plasmid comprises: (a) a polynucleotide encoding a pro-apoptotic protein; and (b) a promoter controlling the expression of the polynucleotide encoding the pro-apoptotic protein. The second plasmid comprises: (a) a polynucleotide sequence encoding an autoantigen or donor antigen; (b) a promoter controlling expression of the polynucleotide sequence encoding the autoantigen or donor antigen; and (c) a plurality of CpG motifs, where the CpG motifs are methylated sufficiently to diminish the recipient's immune response to unmethylated CpG motifs.

In one instance, administration of the plasmids elevates expression of Il-4 (interleukin 4). In another version of this instance, administration of the plasmids further induces a donor-specific tolerogenic response. In yet another version of this instance, administration of the plasmids elevates expression of inhibitory Fc receptor, Fcyllb, and Il-1ra. In still another version of this instance, administration of the plasmids reduces an autoimmune response. In an alternative version of this instance, administration of the plasmids reduces expression of Tnfα (tumor necrosis factor) and Ifnγ (gamma interferon). As will be appreciated by one of skill in the art with reference to the present disclosure, alternative versions of the method can include alternative steps where the first plasmid and the second plasmid are administered simultaneously or sequentially.

### BRIEF DESCRIPTION OF THE DRAWINGS

These features, aspects and advantages of the present invention will become better understood with regard to the following description, appended claims and accompanying drawings where:

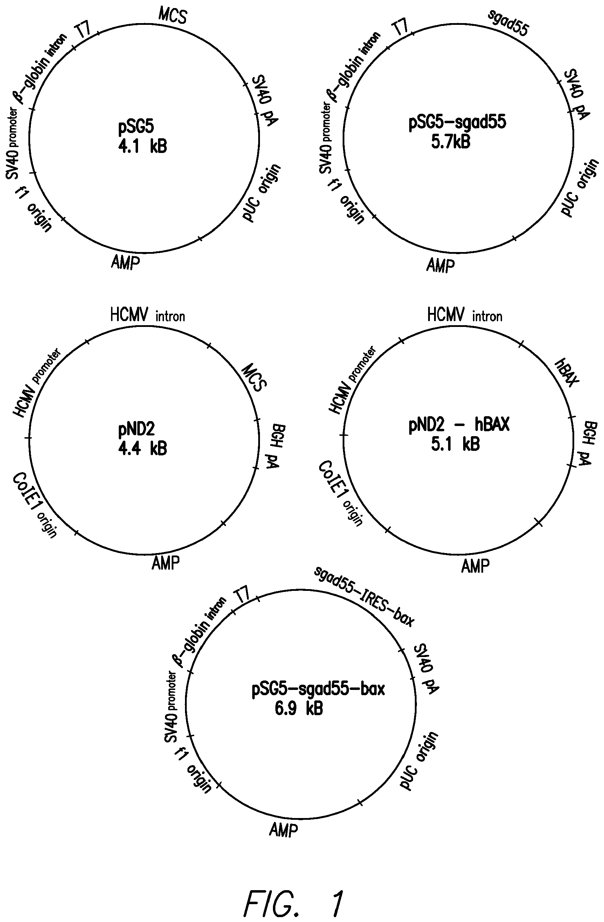

Figure 1 shows schematic depictions of the plasmids disclosed herein, including plasmid vectors pSG5 and pND2, a plasmid containing a polynucleotide encoding secreted glutamic acid decarboxylase 55 (SGAD55) operably linked to a SV40 promoter (pSG5-sgad55), a plasmid containing a polynucleotide encoding a pro-apoptotic protein (hBAX) operably linked to an HCMV promoter (pND2-hBAX), and a plasmid containing a polynucleotide encoding SGAD55 and hBAX operably linked to an SV40 promoter (pSG5-sga55-bax).

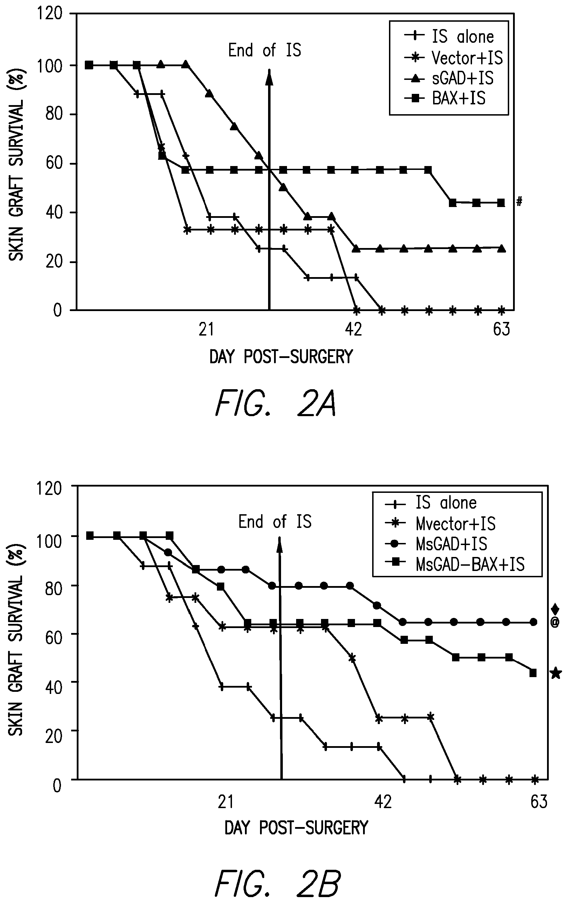

Figure 2 shows the effects of DNA vaccination on skin allograft survival. 7-week-old, age matched C57BL/6 recipients (N= 8-14) received skin grafts from BALB/c donor under minimum immune suppression regimen (IS) that was ended on day 28, and received a weekly i.d. injection of 50 µg of the indicated vaccine. The BAX and sGAD vaccines are non-CpG-methylated plasmid DNA coding for BAX and sGAD alone, respectively. The MsGAD DNA vaccine consists of CpG-methylated plasmid DNA coding for sGAD alone. The MsGAD-BAX vaccine consists of a 4:1 ratio of MsGAD:BAX plasmid DNA. A and B show allograft survival after immunization with non-methylated and methylated vaccines, respectively. #, *P* < 0.003 compared to vector and IS alone (Mann-Whitney), @, *P<* 0.005 compared to methylated vector (Mvector) and IS alone, ◆, *P* < 0.04 compared to sGAD in A, ★, *P* < 0.04 compared to methylated, non-methylated vector controls and IS alone(Kaplan-Meier).

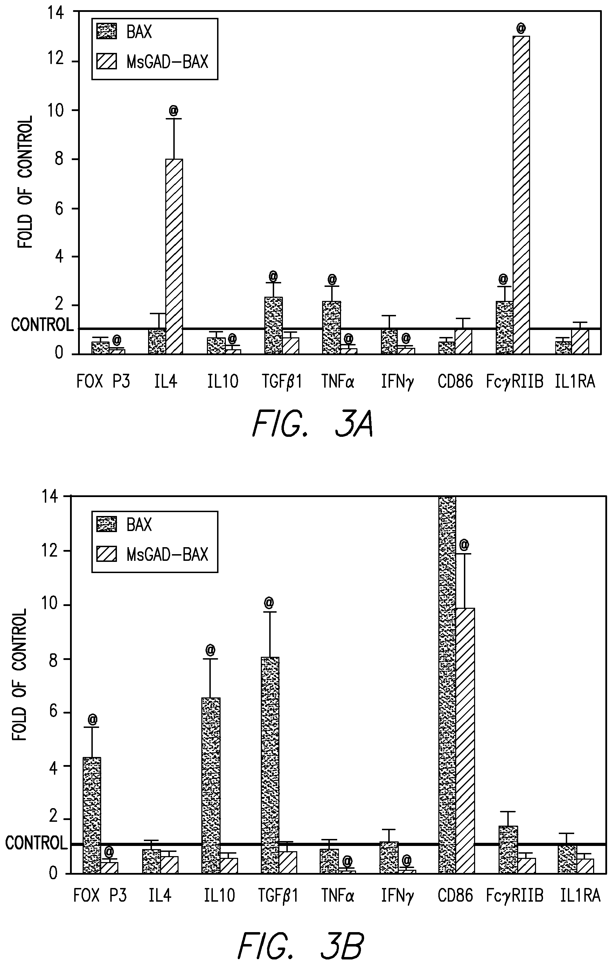

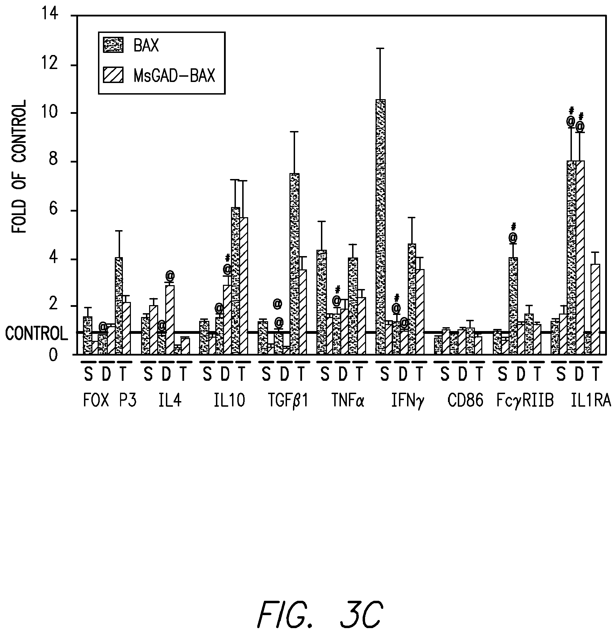

Figure 3 shows the results of quantitative gene expression analysis in skin and LNs of recipient mice. C57BL/6 mice under minimum immunosuppression received BALB/c skin grafts and were immunized with the BAX or MsGAD-BAX DNA vaccine. Fresh skin allografts (A) and fresh lymph nodes (LNs) (B) were taken 2 weeks after transplant for qPCR analysis. In addition, LNs were stimulated with self, donor, or third-party (C3H) antigens for analysis (C). Results are shown as fold of gene expression relative to non-vaccinated C57BL/6 mice under minimum immunosuppression (control). In A and B, @, *P* < 0.05 compared to control. In C, #, @, *P* < 0.05 compared to cells stimulated with self and third-party antigens, respectively.

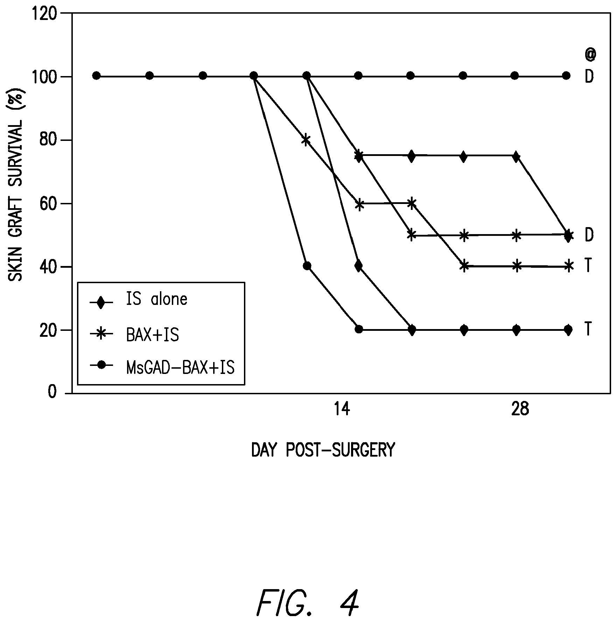

Figure 4 shows the results of the adoptive transfer of immune cells from pooled LNs and spleen. Adoptive transfer donor (C57BL/6) received BALB/c skin graft and minimum immunosuppression. Splenocytes and draining lymph node cells were then isolated on day 14, and injected i.p. on day -2 into adoptive transfer recipients (C57BL/6, N=4-5) receiving donor (BALB/c, D) or third party (C3H, T) skin grafts at day 0. The recipients also received 3 Gy irradiation at day -3, and daily rapamycin i.p. @, *P* < 0.05 compared to third party.

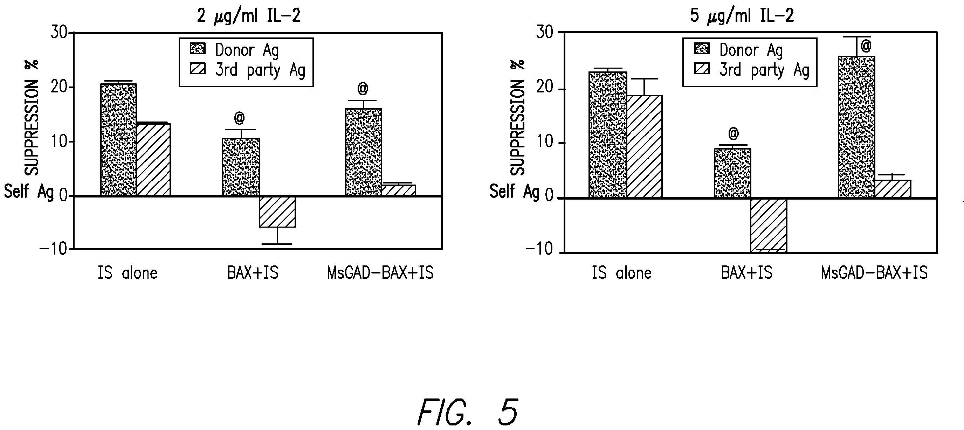

Figure 5 shows the results of mixed lymphocyte reactions where LN cells were isolated from recipients receiving donor skin grafts and immune regimen for 2 weeks, and 4 × 10⁵ LN cells were mixed with 4 × 10⁵ mitomycin treated splenocytes from BALB/c donor or C3H third-party mice in presence of 2 or 5 µg/ml IL-2. @, *P* < 0.05 compared to third party antigens.

### FURTHER DETAILED DESCRIPTION

As used in this disclosure, except where the context requires otherwise, the term "comprise" and variations of the term, such as "comprising," "comprises" and "comprised" are not intended to exclude other additives, components, integers or steps.

As used in this disclosure, the terms "graft" and "transplant" are used interchangeably and refer to an organ or tissue taken from the body and grafted into another area of the same individual or another individual.

As used in this disclosure, the term "allograft" comprises a graft of tissue between individuals of the same species but of disparate genotype; types of donors include cadaveric, living related, and living unrelated.

As used in this disclosure, the term "allogeneic" denotes individuals of the same species but of different genetic constitution.

As used in this disclosure, the term "allogeneic transplantation" denotes transplantation of an allograft.

As used in this disclosure, the term "DNA vaccine" comprises DNA sequences that code for immunogenic proteins located in appropriately constructed plasmids, which include strong promoters, which when injected into an animal are taken up by cells and the immunogenic proteins are expressed and elicit an immune response.

As used in this disclosure, the term "autoantigen" comprises an endogenous antigen that stimulates the production of autoantibodies, as in an autoimmune reaction, as well as part of such endogenous antigens, or modified endogenous antigens that elicit the same response as the full endogenous antigen, as will be understood by those with skill in the art with reference to this disclosure. For example, in the context of this disclosure secreted glutamic acid decarboxylase 55 and humanized BAX are both autoantigens.

As used in this disclosure, the term "donor antigen" comprises an antigen from an allograft that was transplanted into the recipient to take the place of defective or absent cells or tissues, such as for example skin grafts and islet cell transplants, and partial or whole organ transplants, including transplanted hearts, lungs, kidneys and livers, and that stimulates the production of antibodies that produce an immune reaction, as well as part of such donor antigens, or modified donor antigens that elicit the same response as the full donor antigen, as will be understood by those with skill in the art with reference to this disclosure. For example, in the context of this disclosure, secreted glutamic acid decarboxylase 55 is a donor antigen for skin grafts and islet cell transplants.

Examples of a pro-apoptotic protein include BAX (SEQ ID NO:2), a modified caspase, Tumor Necrosis Factor Receptor, Death Receptor 3 (DR3), Death Receptor 4 (DR4), Death Receptor 5 (DR5) and a FAS receptor. As used in this disclosure, the term "hBAX" and "BAX" are interchangeable.

As will be understood by those with skill in the art with reference to this disclosure, when reference is made to a protein encoded by a polynucleotide sequence, the protein includes "conservative substitutions" in which an amino acid is substituted for another amino acid that has similar properties, such that one skilled in the art of peptide 'chemistry would expect the secondary structure and hydropathic nature of the polypeptide to be substantially unchanged. A conservative substitution occurs when one amino acid residue is replaced with another that has a similar side chain. Amino acid residues having similar side chains are known in the art and include families with basic side chains (e.g., lysine (Lys/K), arginine (Arg/R), histidine (His/H)), acidic side chains (e.g., aspartic acid (Asp/Device 400), glutamic acid (Glu/E)), uncharged polar side chains (e.g., glycine (Gly/G), asparagine (Asn/N), glutamine (Gln/Q), serine (Ser/S), threonine (Thr/T), tyrosine (Tyr/Y), cysteine (Cys/C)), nonpolar side chains (e.g., alanine (Ala/A), valine (Val/V), leucine (Leu/L), isoleucine (Ile/I), proline (Pro/P), phenylalanine (Phe/F), methionine (Met/M), tryptophan (Trp/W)), β-branched side chains (e.g., threonine (Thr/T), valine (Val/V), isoleucine (Ile/I)) and aromatic side chains (e.g., tyrosine (Tyr/Y), phenylalanine (Phe/F), tryptophan (Trp/W), histidine (His/H)).

As used in this disclosure, a CpG motif is a polynucleotide region characterized by dinucleotides containing cytosine residues in the sequence CG. As will be understood by those with skill in the art with reference to this disclosure, bacterial DNAs containing unmethylated CpG motifs, stimulate the immune system in mammals to start a sequence of reactions leading to an immune reaction and inflammation. However, methylated CpG motifs may be applied to alleviate or inhibit the unwanted immune stimulation and inflammation by bacterial DNA.

Apoptotic cells are routinely processed by antigen presenting cells (APCs) like dendritic cells (DCs) to establish and maintain antigen-specific tolerance. DNA vaccines can induce apoptotic cells directly *in vivo,* and permit the engineering of the induced apoptotic cells. Unlike the use of immunosuppressants and co-stimulatory blockade, this is a "top down" approach that has the potential to induce specific immunoregulation via physiological modulation of APC function. In some aspects, the present disclosure provides for the use of three different plasmid DNA constructs, and combinations thereof, as DNA vaccines to increase survival of allografts. Specific instances each of the constructs causes increased survival of engrafted skin when injected into mice receiving skin allografts.

According to one instance of the present disclosure, there is provided a method of preventing, delaying the onset of, or treating the rejection of an allograft or an allogeneic transplant. In one instance, the method comprises, first, selecting a recipient in need of a graft or transplant and an allograft or alllogeneic transplant donor. The selection can be made using standard methods as will be understood by those with skill in the art with reference to this disclosure.

According to one instance, the method further comprises engrafting tissue or transplanting a solid organ from the donor to the recipient to take the place of defective or absent cells or tissues. Engrafted tissues or transplanted organs can include a skin grafts, islet cell transplants, and partial or whole organ transplants including transplanted hearts, lungs, kidneys and livers.

According to one instance, the method further comprises administering to the recipient a DNA vaccine comprising one or more polynucleotides encoding (1) a pro-apoptotic protein, (2) an autoantigen or donor antigen, or (3) a pro-apoptotic protein and an autoantigen or donor antigen.

In one instance, a DNA vaccine for use in the present disclosure comprises a plasmid, the plasmid comprising a polynucleotide encoding a pro-apoptotic protein under the control of a promoter capable of expressing the polynucleotide encoding the pro-apoptotic protein.

In another instance, a DNA vaccine for use in the present disclosure comprises a plasmid, the plasmid comprising a polynucleotide encoding an autoantigen or a donor antigen operably linked to a promoter capable of controlling the expression of the polynucleotide encoding the autoantigen or the donor antigen, where the plasmid comprises a plurality of CpG motifs, and where at least some of the plurality of CpG motifs are methylated. In a preferred instance, the CpG motifs are methylated sufficiently to inhibit the recipient's immune response to unmethylated plasmid DNA. In a particularly preferred instance, the plasmid is resistant to digestion by the restriction endonuclease HpaII, which digests unmethylated but not methylated DNA. In another instance, the CpG motifs are methylated by SssI methylase.

In another instance, the plasmid comprising a polynucleotide encoding an autoantigen or a donor antigen can further comprise a polynucleotide encoding a pro-apoptotic protein under the control of a promoter capable of expressing the polynucleotide encoding the pro-apoptotic protein.

In a preferred instance, the promoter capable of expressing the polynucleotide encoding the autoantigen or the donor antigen, and the promoter capable of expressing the polynucleotide encoding the pro-apoptotic protein, are a single promoter.

In a preferred instance, the promoter capable of expressing the polynucleotide encoding the autoantigen or the donor antigen, or the promoter capable of expressing the polynucleotide encoding the pro-apoptotic protein, or both the promoter capable of expressing the polynucleotide encoding the autoantigen or the donor antigen, and the promoter capable of expressing the polynucleotide encoding the pro-apoptotic protein, maintain their promoter function after methylation.

In another instance, the plasmid comprises an internal ribosome entry site (IRES) sequence, to permit translation of the polynucleotide encoding the autoantigen or the donor antigen and the polynucleotide encoding the pro-apoptotic protein from the same transcript.

In another instance, the DNA vaccine of the present disclosure comprises a first plasmid and a second plasmid, or composition comprising a first plasmid and a second plasmid. The first plasmid comprises a polynucleotide encoding an autoantigen or a donor antigen under the control of a promoter capable of expressing the polynucleotide encoding the autoantigen or the donor antigen. The second plasmid comprises a polynucleotide encoding a pro-apoptotic protein under the control of a promoter capable of expressing the polynucleotide encoding the pro-apoptotic protein. The first plasmid comprises a plurality of CpG motifs, and at least some of the plurality of CpG motifs are methylated.

In a preferred instance, the promoter capable of expressing the polynucleotide encoding the autoantigen or the donor antigen maintains its promoter function after methylation. In another instance, the second plasmid comprises a plurality of CpG motifs, and at least some of the plurality of CpG motifs are methylated.

In a preferred instance, the promoter capable of expressing the polynucleotide encoding a pro-apoptotic protein maintains its promoter function after methylation.

In one instance, the DNA vaccine comprises the first plasmid and second plasmid in a ratio of between 1/1000 to 1000/1. In another instance, the composition comprises the first plasmid and second plasmid in a ratio of between 1/100 to 100/1. In another instance, the composition comprises the first plasmid and second plasmid in a ratio of between 1/10 to 10/1.

In one instance of the present disclosure, the recipient is a mammal. In another instance, the recipient is a human.

In another instance, the autoantigen is selected from the group consisting of carbonic anhydrase II, collagen, CYP2D6 (cytochrome P450, family 2, subfamily Device 400, polypeptide 6), glutamic acid decarboxylase, secreted glutamic acid decarboxylase 55, SEQ ID NO:1, insulin, myelin basic protein and SOX-10 (SRY-box containing gene 10).

In another instance, the pro-apoptotic protein is selected from the group consisting of BAX, SEQ ID NO:2, a modified caspase, Tumor Necrosis Factor Receptor, Death Receptor 3 (DR3), Death Receptor 4 (DR4), Death Receptor 5 (DR5) and a FAS receptor.

In a preferred instance, the internal ribosome entry site sequence is an internal ribosome binding site from the EMC virus, SEQ ID NO:3.

The method comprises administering to the recipeint one or more than one dose of a DNA vaccine according to the present disclosure. In a preferred instance, the DNA vaccine is administered in a plurality of doses. In another preferred instance, the dose is between about 0.001 mg/Kg of body weight of the recipient and about 100 mg/Kg of body weight of the recipient. In another preferred instance, the dose is between about 0.01 mg/Kg of body weight of the recipient and about 10 mg/Kg of body weight of the recipient. In another preferred instance, the dose is between about 0.1 mg/Kg of body weight of the recipient and about 1 mg/Kg of body weight of the recipient. In another preferred instance, the dose is about 0.05 mg/Kg of body weight of the recipient. In a preferred instance, the recipient is a human and the dose is between about 0.5 mg and 5 mg. In another preferred instance, the recipient is a human and the dose is between about 1 mg and 4 mg. In another preferred instance, the recipient is a human and the dose is between about 2.5 mg and 3 mg. In another preferred instance, the dose is administered weekly between 2 times and about 100 times. In another preferred instance, the dose is administered weekly between 2 times and about 20 times. In another preferred instance, the dose is administered weekly between 2 times and about 10 times. In another preferred instance, the dose is administered weekly 4 times. In another preferred instance, the dose is administered only once.

Administering the one or more than one dose of a DNA vaccine to the recipient can be accomplished by any suitable route, as will be understood by those with skill in the art with reference to this disclosure. In one instance, administering to the recipient one or more than one dose of a substance or a composition is performed by a route selected from the group consisting of epidermal, intradermal, intramuscular, intranasal, intravenous and oral. In a preferred instance, the DNA vaccine is administered proximal to the site of the allograft or allogeneic transplant. For example, the plasmid DNA does not have to be injected into a skin graft, but can be injected directly intradermally into the recipient.

When the method comprises administering a first plasmid and a second plasmid, the first plasmid and the second plasmid can be administered either sequentially or simultaneously, as will be understood by those with skill in the art with reference to this disclosure.

When the method comprises administering a first plasmid and a second plasmid, the method can further comprise inducing a donor-specific immune response that elevates Th2-like activity, which can include inducing expression of *Il-4* in the allograft.

When the method comprises administering a first plasmid and a second plasmid, the method can further comprise inducing expression of *FcαIIb* in the allograft.

When the method comprises administering a first plasmid and a second plasmid, the method can further comprise decreasing expression of pro-inflammatory Tnf-α and Ifn-γ genes.

In one instance, the method further comprises administering a dose of one or more than one immunosuppressant agent before, on the day of, and/or after engraftment or transplantation.

When the method comprises administering one or more than one immunosuppressant agent, the one or more than one immunosuppressant agent can be administered simultaneously, separately or sequentially.

In one instance, the one or more than one immunosuppressant agent is selected from the group consisting of corticosteroids, glucocorticoids, cyclophosphamide, 6-mercaptopurine (6-MP), azathioprine (AZA), methotrexate cyclosporine, mycophenolate mofetil (MMF), mycophenolic acid (MPA), tacrolimus (FK506), sirolimus ([SRL] rapamycin), everolimus (Certican), mizoribine, leflunomide, deoxyspergualin, brequinar, azodicarbonamide, vitamin D analogs, such as MC1288 and bisindolylmaleimide VIII, antilymphocyte globulin , antithymocyte globulin (ATG), anti-CD3 monoclonal antibodies, (Muromonab-CD3, Orthoclone OKT3), anti-interleukin (IL)-2 receptor (anti-CD25) antibodies, (Daclizumab, Zenapax, basiliximab, Simulect), anti-CD52 antibodies, (Alemtuzumab, Campath-1H), anti-CD20 antibodies (Rituximab, Rituxan), anti-tumor necrosis factor (TNF) reagents (Infliximab, Remicade, Adalimumab, Humira) and LFA-1 inhibitors (Efalizumab, Raptiva) .

The dosages of the immunosuppressant agents will vary depending on the individual to be treated, the route of administration, and the nature and severity of the condition to be treated. For example, an initial dose of about 2 to 3 times the maintenance dose may suitably be administered about 4 to 12 hours before transplantation, followed by a daily dosage of 2 to 3 times the maintenance dose for one to two weeks, before gradually tapering down at a rate of about 5% a week to reach the maintenance dose.

The skilled person may determine those dosages that provide a therapeutic amount of an immunosuppressant agent at a level that is tolerated. In a preferred instance, the method further comprises administering a single dose of antilymphocyte globulin, of about 1.6 mg/20g of body weight on the day of engraftment or transplantation. In another preferred instance, rapamycin may be applied at a dosage range of from about 0.05 to about 15 mg/kg/day, more preferably from about 0.25 to about 5 mg/kg/day and most preferably from about 0.5 to about 1.5 mg/kg/day. Ideally, the administration of doses of one or more than one immunosuppressant agent can be curtailed after effective treatment with the DNA vaccine.

In one instance, the method further comprises, after administering the DNA vaccine, monitoring the recipient for rejection of the allograft of transplant. In a preferred instance, the recipient is monitored for rejection of the allograft or transplant after tapering off or discontinuing the administration of immunosuppressant agents.

### EXAMPLE 1

### Plasmid DNA constructs, methylation, and amplification

The following examples were designed to test whether our pro-apoptotic DNA vaccination strategy is applicable to prevention of solid allograft rejection. Our model suggests that injection of plasmid DNA coding for BAX near the allograft would cause recruitment of APCs after induction of apoptotic cells, the APCs would process donor antigens under tolerogenic conditions, and a protective, immunoregulatory response would be induced.

In order to compare the efficacy of different DNA vaccines for preventing, delaying the onset of or treating the rejection of an allograft or organ transplant according to the present invention, several plasmids were prepared. Referring now to Figure 1, there are shown, respectively, a schematic depiction of pSG5, pND2, pSG5-SGAD55, and pND2-hBAX.

Plasmid pSG5 was purchased from Stratagene (San Diego, CA US). The remaining plasmids were produced using standard techniques. Plasmid pND2-BAX carries a BAX cDNA under transcriptional control of the CMV promoter, and plasmid pSG5-SGAD55 carries a cDNA construct encoding a secreted form of GAD65 under transcriptional control of the SV-40 promoter.

With reference to Figure 1, the abbreviations shown are standard, as will be understood by those with skill in the art with reference to this disclosure, including: AMP (ampicillin resistance gene for selection in *E. coli*); BGH pA (bovine growth hormone polyadenylation sequence); ColE1 origin (origin of replication in *E. coli*); f1 origin (origin of replication for filamentous phage f1 to generate single stranded DNA); hBAX (human bax cDNA), SEQ ID NO: 2; HCMV promoter (promoter from cytomegalovirus); HCMV intron (intron from cytomegalovirus); MCS (multiple cloning site); pUC origin (origin of replication for *E. coli* from pUC plasmid); sgad55 (secreted GAD cDNA construct), SEQ ID NO:1; SV40 promoter (simian virus 40 promoter); SV40 pA (simian virus 40 polyadenylation sequence); and T7 (T7 promoter).

Plasmids pSG5 and pSG-SGAD55 were methylated to produce methylated pSG5, and methylated pSG5-SGAD55, by amplification in *E.coli* strain ER1821 carrying a plasmid encoding the *Sssl* methylase ( New England Biolabs, Beverly, MA US). *SssI* methylates the dinucleotide motif CpG in DNA in a manner corresponding to mammalian methylases by covalently adding a single methyl group to the dinucleotide motif CpG.

Plasmid DNA was isolated after amplification in *Escherichia coli* strain DH5α or ER1821 using the Endofree Plasmid DNA Purification Kit (Qiagen, Valencia, CA). Successful methylation was confirmed by digesting the isolated plasmid DNA with the restriction enzyme *HpaII* which digests unmethylated but not methylated DNA, where resistance to *HpaII* digestion indicates successful methylation.

### EXAMPLE 2

### Skin Allograft Survival

An allograft skin transplant model was used as proof of principle because it is one of the most difficult models for prevention of organ rejection. A combination of two of the plasmid constructs (2-plasmid vaccine coding for secreted GAD, i.e., SGAD55, and pro-apoptotic BAX, with one plasmid methylated with SssI methylase) has been used successfully as a DNA vaccine for therapy of type 1 diabetes in NOD mice. However, each of the two DNA constructs (plasmid DNA coding for BAX alone, or SssI-methylated plasmid DNA coding for SGAD55 alone) were found to be ineffective for therapy of diabetes on their own. Unexpectedly, we now find that all three alternatives can prevent skin allograft rejection.

The effects of i.d. injection of different DNA vaccines were investigated in C57BL/6 mice receiving minimum immunosuppression and skin allografts from BALB/c mice. 0.7 × 0.7 cm full-thickness back skin grafts from BALB/c donors or C3H third party were transplanted onto the back of C57BL/6 recipients. With the exception of the untreated group, anti-lymphocyte immunoglobulin ALG (1.6 mg/20g BW) was given i.p. once on day 0. Fifty µg of the following plasmid DNAs: 1) vector alone, 2) DNA coding for BAX alone, 3) *SssI*-methylated DNA coding for SGAD55 alone (Msgad55), or 4) DNA coding for BAX together with *SssI*-methylated DNA coding for SGAD55 (Msgad55+bax), were injected i.d. near the skin graft on day 0, 3, 7, and then weekly. Rapamycin (1 mg/kg) was injected i.p. daily from days 0-27. Bandages were removed on day 10, and skin graft rejection was defined as 85% loss of the graft area, and confirmed by pathological analysis.

Figure 2A shows that non-methylated plasmid DNA coding for BAX alone could significantly delay skin rejection compared to mice receiving vector DNA alone, but that non-methylated plasmid DNA coding for sGAD alone did not. Injection of non-methylated plasmid vector alone had no significant effect on allograft survival compared to non-vaccinated, immunosuppressed mice.

In addition, we investigated the effects of CpG-methylation of vaccine DNA and of combined delivery of plasmid DNA coding for sGAD and BAX on skin allograft survival. CpG-methylation of the vaccine coding for sGAD alone (MsGAD) resulted in increased allograft survival (Fig. 2B), which was significant compared to mice immunized with the non-methylated vaccine coding for sGAD alone and CpG-methylated vector control. However, combined delivery of CpG-methylated plasmid DNA coding for secreted GAD and plasmid DNA coding for BAX resulted in increased survival only when compared with mice vaccinated with vector controls and non-immunized mice, which indicated an antagonistic effect of BAX.

Without being held to any particular underlying mechanisms behind the observed effects, we suspect that it may involve donor antigen-specific immunoregulation for the constructs coding for BAX, based on our previous model for a pro-apoptotic DNA vaccination strategy. We do not know what the mechanism of action is for the third construct (SssI-methylayed DNA coding for SGAD55).

### EXAMPLE 3

### RNA isolation and qPCR

We investigated the effects of the MsGAD-BAX vaccine, which showed efficacy in both prevention of skin allograft rejection and amelioration of new-onset diabetes in NOD mice in previous work, and of the BAX vaccine on the expression of chosen genes in transplanted BALB/c skin and freshly isolated LNs of recipient C57BL/6 mice.

For immune analysis, draining lymph nodes of mice receiving ALG and rapamycin alone, and from mice treated with the vaccine coding for BAX and SGAD55, were taken 2 weeks after transplantation. Lymph nodes were cultured in the presence of inactivated splenocytes from C57/BL6 (recipient), BALB/c (donor), or DBA (third party) as sources of antigens, and CD4+CD25+ and CD4+CD25- cells were isolated for proliferation assays.

Quantitative PCR analysis of expression of selected genes was performed with skin allografts, cultured lymph nodes, and CD4+CD25+ /CD25-cells isolated from proliferation assays. In addition to the *Il-4*, *Il-10*, *Tgf-β1*, *Tnf-α*, and *Ifn-γ* genes, we quantified the expression of genes coding for the co-stimulatory molecules CD80 and CD86 found on APCs, the transcriptional factor FOXP3 synthesized by regulatory T cells (Tregs), and the inhibitory receptor FcγRIIB and IL-1-antagonist IL-1RA cytokine, which are both up-regulated in murine tolerogenic DCs.

Total RNA was isolated using Trizol LS reagent (Invitrogen, Cartsbad, CA) from freshly taken skin allograft and draining LNs 2 weeks after transplantation. RNA was also isolated from LNs cultured for 3 days in as described above. qPCR was performed using the iCycler system and SYBR green (Bio Rad, Hercules, CA) with 200 ng of total RNA as template and primers specific for the chosen cDNAs, and for the GAPDH cDNA as a housekeeping gene.

In the transplanted skin, the most striking differences between the two vaccines were increased expression of the *Il-4* and *FcγrIIb* genes and decreased expression of the *Tnf-α* and *Ifn-γ* genes in mice immunized with MsGAD-BAX (Fig. 3A). In LNs, the most apparent differences were increased expression of the *Foxp3, II-10, Tgf-β1*, and *Cd86* genes in mice immunized with BAX, and decreased expression of the *Tnf*-*α* and *Ifn-γ* genes in mice immunized with MsGAD-BAX (Fig. 3B). Increased expression of *Cd86* was also observed in mice immunized with MsGAD-BAX, but to an extent that was proportional to the amount of delivered *Bax* cDNA. No significant change in the expression of the *Cd80* gene was observed (data not shown). These data indicated that the two vaccines induced clearly distinct immune responses.

Several pieces of evidence indicated that BAX and MsGAD-BAX induced donor-specific immune responses. First, gene expression analysis of LNs from C57BL/6 mice receiving BALB/c skin allografts and cultured for 3 days revealed several significant differences when cells were stimulated with self, donor, or third party antigens (Fig. 3C). Compared to stimulation with third party antigens, cells from mice immunized with BAX and MsGAD-BAX and stimulated with donor antigens showed a significant change in expression in 8 and 5 of the 9 chosen genes, respectively. In contrast, compared to stimulation with self antigens, cells from mice immunized with BAX and MsGAD-BAX and stimulated with donor antigens showed change in expression in 4 and 2 of the 9 genes, respectively. These results indicated that the gene expression profile after donor antigen stimulation was markedly different from the third party antigen profile and more similar to the self antigen profile. In addition, the finding that genes like *FcγrIIb* and *Il-1ra*, which are up-regulated in tolerogenic DCs, were induced only when stimulated with donor antigens corroborated the notion of a donor-specific tolerogenic response.

### EXAMPLE 4

### Adoptive cell transfer

Cells from spleen and draining axillary and cervical lymph nodes (LNs) were isolated 2 weeks post donor skin graft transplant and suspended in PBS; 5 × 10⁶ total cells per recipient were injected i.p. at day -2, and 3 Gy TBI irradiation was given on day -3. Donor or third party back skin grafts were transplanted on day 0, and rapamycin (1 mg/kg) was given i.p. daily.

Results from adoptive transfer experiments indicated that splenocytes and LN cells from C57BL/6 mice receiving BALB/c skin graft and immunized with MsGAD-BAX could transfer survival of donor, but not third party graft (Fig. 4). In contrast, cells from mice immunized with BAX did not significantly prevent rejection of either donor or third party graft.

### EXAMPLE 5

### MLR

LNs were dispersed and stimulated with mitomycin C-treated splenocytes from C57BL/6, BALB/c, and C3H mice for 5 days with 2 or 5 µg/ml IL-2 for MLR using 2 µM CFSE-labeled LN cells. Results from MLR using CSFE-labeled LN cells cultured with inactivated splenocytes from donor and third party antigens also indicated a donor-specific immune response for each vaccine. In the presence of 2 µg/ml IL-2, LN cells from non-vaccinated, immunosuppressed mice did not show a significant difference in suppression when stimulated with donor or third party antigens (Fig. 5A). In contrast, LN cells from mice immunized with BAX or MsGAD-BAX showed a significant difference in their response to third party antigen compared to donor antigen. Culture with 5 µg/ml IL-2 did not restore proliferation and accentuated these differences (Fig. 5B).

### Conclusion

Specific instances of the present disclosure described herein address the problems of preventing rejection of allografts without having to know the identity of the donor antigens, and of reducing the need for immunosuppressants that are known to have serious side-effects over the long term. Our results indicate that "pro-apoptotic" DNA vaccination can be applied successfully to solid organ transplantation. Injection of BAX DNA appears to induce a donor-specific immunoregulatory response that contributes to increased graft survival.

One explanation for the observed effects is that vaccination with plasmid DNA coding for BAX induces apoptotic cells that recruit antigen presenting cells . The antigen presenting cells process the tolerogenic apoptotic cells together with donor antigens and protects the allograft, most likely via multiple immune mechanisms. However, considering that expression of *Foxp3, Il-10 and Tgf-β1* was consistently higher in non-vaccinated, immunosuppressed animals, other mechanisms of tolerance induced by our DNA vaccination strategy are likely to play a role in expressing graft survival. Unexpectedly, injection of *SssI*-methylated coding for SGAD55 alone could prolong graft survival. The same vaccine was ineffective for treatment of type diabetes in our previous studies. Consequently, the underlying mechanism for graft survival using this approach remains to be determined.

Our data indicates that both the BAX and MsGAD-BAX vaccines induced a donor-specific immune response, albeit via two different mechanisms. The BAX vaccine caused changes in gene expression mainly in fresh LNs, most likely because of the recruitment of APCs to LNs after plasmid DNA-mediated induction of apoptosis. Increased expression of *Cd86* associated with delivery of the *bax* cDNA was not linked with concomitant *Cd80* expression, which indicated induction of *Cd86.* The *CD86* molecule is the main ligand for *CD28* and promotes inflammation. Significantly, LN cells of BAX-immunized mice showed highly increased expression of the *Tnf-α* and *Ifn-γ* genes when stimulated with self antigens, which was not observed with cells stimulated with donor antigens or with cells from mice immunized with MsGAD-BAX and stimulated with self antigens. These results suggest that induction of recipient apoptotic cells near the allograft could have amplified the autoimmune response that is known to be induced by a skin allograft via an indirect alloresponse.

In addition, fresh LNs and LN cells from BAX-immunized mice stimulated with third party antigen showed increased expression of *Foxp3, Il-10*, and *Tgf-β1*, which is associated with CD4⁺CD25⁺ regulatory T cell activity. However, it is unlikely that such cells were responsible alone for increased allograft survival, because adoptive transfer of spleen and LN cells from BAX-immunized mice did not prevent rejection of either donor or third party allografts. Rather, these results suggested that the BAX vaccine promoted graft survival via a different mechanism, like clonal deletion. The lesser efficacy of the BAX vaccine could have been the result of the autoimmune response it induced. Nevertheless, it the efficacy of the vaccine may be improved using CpG-methylation of plasmid DNA to lower inflammation resulting from interaction between bacterial plasmid DNA and TLR-9, and by injecting the DNA outside of the inflammatory milieu induced by the allograft.

In contrast with BAX, the MsGAD-BAX vaccine induced expression in the allograft of *Il-4*, which indicated Th2-like activity, and of *FcγIIb*, which is up-regulated in tolerogenic DCs. Moreover, the MsGAD-BAX vaccine caused decreased expression of the pro-inflammatory *Tnf-α* and *Ifn-γ* genes in both transplanted skin and fresh LNs, did not appear to promote autoimmunity, and induced cells that transferred graft survival in a donor-specific manner. Notably, GAD is found in skin, which we confirmed in skin allografts using qPCR (data not shown), and is up-regulated in inflamed tissues. Therefore, the sGAD polypeptide may act as a regulatory autoantigen that prevents allograft rejection. Indeed, the strongest evidence that autoimmunity plays a role in allograft rejection comes from experiments where administration of autoantigens present in the donor graft can prevent rejection of lung or heart allografts, or accelerate rejection when injected with Freund's adjuvant into recipient. Our finding that *Il-4* expression was increased in skin allograft corroborates the notion that this cytokine can be a key element in the process.

In conclusion, plasmid DNA-mediated induction of recipient apoptotic cells and delivery of an allograft-associated autoantigen form the basis of a new DNA vaccination strategy that targets allograft-induced autoimmunity to prevent transplant rejection. This approach, which is readily amenable to manipulation at the molecular and cellular levels for further improvement, provides a promising means to control chronic rejection in which allograft-induced autoimmunity is thought to play a significant role.

## Claims

1. A DNA vaccine for use in preventing, delaying the onset of, or treating rejection of a skin allograft or a skin allogeneic transplant, wherein the DNA vaccine comprises (a) a first plasmid comprising a polynucleotide encoding secreted glutamic acid decarboxylase 55 (sGAD55) under the control of a promoter which expresses the polynucleotide encoding sGAD55, and comprising a plurality of CpG motifs, where the CpG motifs are methylated sufficiently to diminish the recipient's immune response to unmethylated CpG motifs, and wherein at least some of the plurality of CpG motifs are methylated and resistant to digestion by restriction endonuclease *Hpa*II, and (b) a second plasmid comprising a polynucleotide encoding Bax under the control of a promoter which expresses the polynucleotide encoding BAX; and wherein the use comprises:
(i) selecting a recipient in need of a graft or transplant and an allograft or allogeneic transplant donor;
(ii) grafting tissue or transplanting skin from the donor to the recipient; and
(iii) intradermally administering to the recipient one or more than one dose of the DNA vaccine.

2. The DNA vaccine for use of claim 1, where the DNA vaccine is administered by an injection proximal to the site of the skin allograft or skin allogeneic transplant.

3. The DNA vaccine for use of any one of claims 1 or 2, wherein the DNA vaccine is administered in a plurality of doses.

4. The DNA vaccine for use of any one of claims 1-3, wherein the dose is administered weekly between two times and 100 times.

5. The DNA vaccine for use of any one of claims 1-4, wherein the DNA vaccine comprises the first plasmid and the second plasmid in a ratio of 1/10 to 10/1.

6. The DNA vaccine for use of any one of claims 1-5, wherein the first plasmid is methylated with an *Sss*I DNA methyltransferase.

7. The DNA vaccine for use of any one of claims 1-6, further comprising administering a dose of one or more than one immunosuppressant agent before, on the day of, and/or after engraftment or transplantation.

8. The DNA vaccine for use of claim 7, wherein the dose of one or more than one immunosuppressant agent is administered simultaneously, separately or sequentially with the DNA vaccine.

9. The DNA vaccine for use of claim 7 or 8, wherein the one or more than one immunosuppressant agent is selected from corticosteroids, glucocorticoids, cyclophosphamide, 6-mercaptopurine, azathioprine, methotrexate cyclosporine, mycophenolate mofetil, mycophenolic acid, tacrolimus, sirolimus, everolimus, mizoribine, leflunomide, deoxyspergualin, brequinar, azodicarbonamide, vitamin D analogs, antilymphocyte globulin, antithymocyte globulin, anti-CD3 monoclonal antibodies, anti-interleukin-2 receptor (anti-CD25) antibodies, anti-CD52 antibodies, anti-CD20 antibodies, anti-tumor necrosis factor reagents and LFA-1 inhibitors.

10. The DNA vaccine for use of claim 9, comprising:
administering a single dose of antilymphocyte globulin, at a dosage of 1.6 mg/20g of body weight, on the day of engraftment or transplantation; or
administering rapamycin at a dosage of from 0.05 to 15 mg/day.

## Patentansprüche

1. DNA-Impfstoff zur Verwendung bei der Prävention, der Verzögerung des Beginns oder der Behandlung der Abstoßung eines Haut-Allografts oder einer Haut-Allotransplantation, wobei der DNA-Impfstoff Folgendes umfasst: (a) ein erstes Plasmid, das ein Polynukleotid umfasst, das für sezernierte Glutaminsäuredecarboxylase 55 (sGAD55) kodiert, unter der Steuerung eines Promotors, der das Polynukleotid, das für sGAD55 kodiert, exprimiert, und das eine Vielzahl von CpG-Motiven umfasst, wobei die CpG-Motive genügend methyliert sind, um die Immunantwort des Empfängers auf unmethylierte CpG-Motive zu vermindern, und wobei mindestens einige von der Vielzahl von CpG-Motiven methyliert und resistent gegen Verdau durch Restriktionsendonuklease *Hpa*II sind und (b) ein zweites Plasmid, das ein Polynukleotid umfasst, das für Bax kodiert, unter der Steuerung eines Promotors, der das Polynukleotid, das für BAX kodiert, exprimiert; und wobei die Verwendung Folgendes umfasst:
(i) Auswählen eines Empfängers mit Bedarf an einem Graft oder Transplantat und eines Allograft- oder Allotransplantat-Spenders;
(ii) Transplantieren von Gewebe oder Haut des Spenders in den Empfänger; und
(iii) intradermales Verabreichen einer oder mehr als einer Dosis des DNA-Impfstoffs an den Empfänger.

2. DNA-Impfstoff zur Verwendung nach Anspruch 1, wobei der DNA-Impfstoff mittels einer Injektion proximal zur Haut-Allograft- oder Haut-Allotransplantatstelle verabreicht wird.

3. DNA-Impfstoff zur Verwendung nach einem der Ansprüche 1 oder 2, wobei der DNA-Impfstoff in einer Vielzahl von Dosen verabreicht wird.

4. DNA-Impfstoff zur Verwendung nach einem der Ansprüche 1-3, wobei die Dosis wöchentlich zwischen zweimal und 100-mal verabreicht wird.

5. DNA-Impfstoff zur Verwendung nach einem der Ansprüche 1-4, wobei der DNA-Impfstoff das erste Plasmid und das zweite Plasmid in einem Verhältnis von 1/10 bis 10/1 umfasst.

6. DNA-Impfstoff zur Verwendung nach einem der Ansprüche 1-5, wobei das erste Plasmid mit einer Sss-DNA-Methyltransferase methyliert ist.

7. DNA-Impfstoff zur Verwendung nach einem der Ansprüche 1-6, die weiter die Verabreichung einer Dosis von einem oder mehr als einem Immunsuppressivum vor dem Engraftment oder der Transplantation, am Tag des Engraftments oder der Transplantation und/oder nach dem Engraftment oder der Transplantation umfasst.

8. DNA-Impfstoff zur Verwendung nach Anspruch 7, wobei die Dosis von einem oder mehr als einem Immunsuppressivum gleichzeitig mit dem DNA-Impfstoff, getrennt oder sequenziell verabreicht wird.

9. DNA-Impfstoff zur Verwendung nach Anspruch 7 oder 8, wobei das eine oder mehr als eine Immunsuppressivum aus Corticosteroiden, Glucocorticoiden, Cyclophosphamid, 6-Mercaptopurin, Azathioprin, Methotrexat, Cyclosporin, Mycophenolat-Mofetil, Mycophenolsäure, Tacrolimus, Sirolimus, Everolimus, Mizoribin, Leflunomid, Desoxyspergualin, Brequinar, Azodicarbonamid, Vitamin D-Analoga, Antilymphozytenglobulin, Antithymozytenglobulin, monoklonalen Anti-CD3-Antikörpern, Anti-Interleukin-2-Rezeptor-Antikörpern (Anti-CD25-Antikörpern), Anti-CD52-Antikörpern, Anti-CD20-Antikörpern, Anti-Tumor-Nekrose-Faktor-Reagenzien und LFA-1-Inhibitorenausgewählt ist.

10. DNA-Impfstoff zur Verwendung nach Anspruch 9, umfassend:
Verabreichung einer Einzeldosis von Antilymphozytenglobulin, in einer Dosierung von 1,6 mg/20 g Körpergewicht, am Tag des Engraftments oder der Transplantation; oder
Verabreichung von Rapamycin in einer Dosierung von 0,05 bis 15 mg/Tag.

## Revendications

1. Vaccin à ADN destiné à une utilisation dans la prévention, le retardement de l'apparition ou le traitement du rejet d'une allogreffe cutanée ou d'une transplantation allogénique cutanée, le vaccin à ADN comprenant (a) un premier plasmide comprenant un polynucléotide codant l'acide glutamique décarboxylase 55 sécrété (sGAD55) sous le contrôle d'un promoteur qui exprime le polynucléotide codant sGAD55, et comprenant une pluralité de motifs CpG, les motifs CpG étant suffisamment méthylés pour diminuer la réponse immunitaire du receveur aux motifs CpG non méthylés, et dans lequel au moins certains de la pluralité de motifs CpG sont méthylés et résistants à la digestion par l'endonucléase de restriction *Hpa*II*,* et (b) un deuxième plasmide comprenant un polynucléotide codant Bax sous le contrôle d'un promoteur qui exprime le polynucléotide codant BAX ; et l'utilisation comprenant :
(i) la sélection d'un receveur ayant besoin d'une greffe ou d'une transplantation et d'un donneur d'allogreffe ou de transplantation allogénique ;
(ii) une greffe de tissu ou une transplantation cutanée du donneur au receveur ; et
(iii) l'administration au receveur par voie intradermique d'une ou plusieurs doses du vaccin à ADN.

2. Vaccin à ADN destiné à une utilisation selon la revendication 1, le vaccin à ADN étant administré par une injection proximale au site de l'allogreffe cutanée ou de la transplantation allogénique cutanée.

3. Vaccin à ADN destiné à une utilisation selon l'une quelconque des revendications 1 ou 2, le vaccin à ADN étant administré en une pluralité de doses.

4. Vaccin à ADN destiné à une utilisation selon l'une quelconque des revendications 1 à 3, dans lequel la dose est administrée une fois par semaine entre 2 fois et 100 fois.

5. Vaccin à ADN destiné à une utilisation selon l'une quelconque des revendications 1 à 4, le vaccin à ADN comprenant le premier plasmide et le deuxième plasmide selon un rapport de 1/10 à 10/1.

6. Vaccin à ADN destiné à une utilisation selon l'une quelconque des revendications 1 à 5, dans lequel le premier plasmide est méthylé avec une ADN méthyltransférase *Sss*I.

7. Vaccin à ADN destiné à une utilisation selon l'une quelconque des revendications 1 à 6, comprenant en outre l'administration d'une dose d'un ou plusieurs agents immunosuppresseurs avant, le jour de, et/ou après la greffe ou la transplantation.

8. Vaccin à ADN destiné à une utilisation selon la revendication 7, dans lequel la dose d'un ou plusieurs agents immunosuppresseurs est administrée simultanément, séparément ou séquentiellement avec le vaccin à ADN.

9. Vaccin à ADN destiné à une utilisation selon la revendication 7 ou 8, dans lequel lesdits un ou plusieurs agents immunosuppresseurs sont sélectionnés parmi des corticostéroïdes, des glucocorticoïdes, le cyclophosphamide, la 6-mercaptopurine, l'azathioprine, la combinaison méthotrexate-cyclosporine, le mycophénolate mofétil, l'acide mycophénolique, le tacrolimus, le sirolimus, l'évérolimus, la mizoribine, le leflunomide, la désoxyspergualine, le brequinar, l'azodicarbonamide, des analogues de la vitamine D, la globuline antilymphocytaire, la globuline d'antithymocytaire, des anticorps monoclonaux anti-CD3, des anticorps anti-récepteur de l'interleukine-2 (anti-CD25), des anticorps anti-CD52, des anticorps anti-CD20, des réactifs anti-facteur de nécrose tumorale et des inhibiteurs de LFA-1.

10. Vaccin à ADN destiné à une utilisation selon la revendication 9, comprenant :
l'administration d'une dose unique de globuline antilymphocytaire, selon une posologie de 1,6 mg/20 g de poids corporel, le jour de la greffe ou de la transplantation ; ou
l'administration de rapamycine selon une posologie de 0,05 à 15 mg/jour.
